# EUROPEAN PATENT SPECIFICATION

(11) **EP 1 583 485 B1**
(45) Date of publication and mention of the grant of the patent: **21.09.2011**
(21) Application number: 04701149.9
(22) Date of filing: 09.01.2004
(51) Int. Cl.: A61F 2/01, A61B 17/32

(54) **SNARE RETRIEVABLE EMBOLIC PROTECTION FILTER WITH GUIDEWIRE STOPPER**
MIT SCHLINGE ZURÜCKHOLBARER EMBOLIESCHUTZFILTER MIT FÜHRUNGSDRAHTANSCHLAG
FILTRE DE PROTECTION EMBOLIQUE RECUPERABLE A L'AIDE D'UN COLLET, EQUIPE D'UN OBTURATEUR DE GUIDE-FIL

(30) Priority: 14.01.2003 US 341667
(43) Date of publication of application: 12.10.2005
(73) Proprietor: Boston Scientific Limited, St. Michael (BB)
(72) Inventor: ESKURI, Alan, D, Hanover, MN 55341 (US); VRBA, Anthony, C, Maple Grove, MN 55311 (US)
(74) Representative: Vossius & Partner
(86) International application number: PCT/US2004/000384
(87) International publication number: WO 2004/064679

(56) References cited:
- WO-A-01/56484
- WO-A-98/23322
- US-A1- 2002 116 024
- US-A1- 2002 121 472
- US-A1- 2002 193 827
- US-B1- 6 436 121

## Description

The present invention relates generally to the field of intravascular filters. More specifically, the present invention pertains to retrieval mechanisms for embolic protection filters.

Distal protection devices such as embolic protection filters are generally placed within a body lumen, such as an artery or vein, downstream of a site where a therapeutic device will be used. Examples of procedures employing such filters include angioplasty, atherectomy, thrombectomy, and stent placement These procedures typically involve percutaneously inserting and delivering within the artery or vein, a guidewire and embolic protection filter to a location distal a coronary lesion. Once in place, a therapeutic device such as an angioplasty or atherectomy catheter can be advanced to the site of the lesion to treat the vessel. During the procedure, embolic debris such as plaque or thrombus become dislodged from the vessel wall, and flow downstream where they are collected by the filter. After the procedure is performed, the therapeutic device, filter and captured embolic debris can be removed from the body.

US 6 436 121 B1 discloses a blood filter comprising a plurality of central struts, the distal ends thereof intersecting at a vertex, a hollow tubular member having distal and proximal ends in fluid communication, and having at least one vertical strut extending therefrom, and means for retaining the plurality of central struts within the hollow tubular member.

WO 01/56484 A1 is directed to a retrieval device or snare for grasping foreign particles and retrieving them from the body of a patient. The retrieval device includes a catheter open at both ends such that it may be advanced along a guidewire. The distal end of a wire is attached to the first catheter near an opening system within the catheter. The opening system may be a slot or a pair of holes. A portion of the wire is oriented external of the catheter. Manipulation of the proximal end of the wire causes a portion of the wire to form a loop external of the catheter. The catheter may be tapered, as may be the wire. A reinforcing material may be attached to the portion of the wire that forms the loop. Another reinforcing material may also be attached to the proximal portion of the wire in order to stiffen the wire and improve its pushability.

The present invention relates to medical retrieval devices for retrieving embolic protection filters within a vessel according to claim 1. The retrieval device includes an embolic protection filter slidably disposed along a guidewire, and a snare retrieval catheter adapted to ensnare the embolic protection filter. The embolic protection filter may comprise a filter frame, a plurality of expandable struts, and a filter mesh or membrane operatively coupled to the expandable struts. A radial notch formed on the proximal portion of the filter frame forms a collar that can be utilized by the snare retrieval catheter to retrieve the embolic protection filter from the vessel.

The snare retrieval catheter comprises an elongated tubular member having a first lumen configured to slidably receive the guidewire, and a second lumen configured to receive an elongated member that can be actuated within the catheter to retrieve the embolic protection filter. The elongated member may include a flexible wire that extends through the second lumen of the elongated tubular member, forming a loop portion distal the distal end of the snare retrieval catheter. The size of the loop can be adjusted to ensnare the radial notch formed on the proximal portion of the embolic protection filter.
Figure 1 is a perspective view of a filter retrieval system in accordance with an exemplary embodiment of the present invention;
Figure 2 is a cross-sectional view of the snare retrieval catheter illustrated in Figure 1, wherein the catheter is shown along line 2-2;
Figure 3 is a plan view illustrating a guidewire inserted in a vessel distal a lesion;
Figure 4 is a plan view of a distal stop advanced along the guidewire illustrated in Figure 3;
Figure 5 is a plan view of an embolic protection filter advanced along the guidewire and abutting the distal stop illustrated in Figure 4;
Figure 6 is a plan view of a sheath advanced about the embolic protection filter; and
Figure 7 is a plan view of a snare retrieval catheter advanced along the guidewire and ensnaring the collapsed embolic protection filter.

The following description should be read with reference to the drawings, in which like elements in different drawings are numbered in like fashion. The drawings, which are not necessarily to scale, depict selected embodiments and are not intended to limit the scope of the invention. Although examples of construction, dimensions, and materials are illustrated for the various elements, those skilled in the art will recognize that many of the examples provided have suitable alternatives that may be utilized.

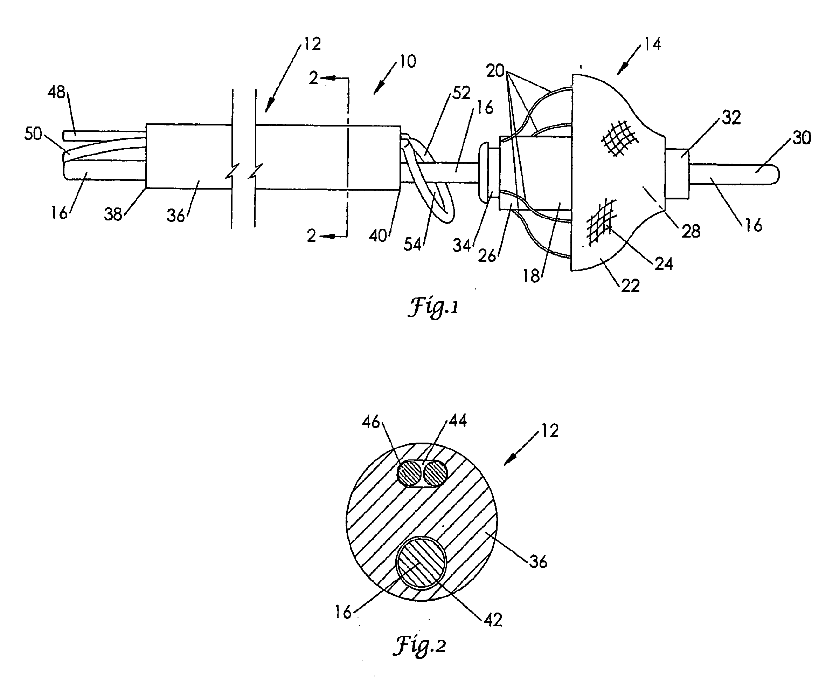

Figure 1 is a perspective view of an exemplary embodiment of a filter system for retrieving an embolic protection filter within a blood vessel Filter system 10 comprises a snare retrieval catheter 12, and an embolic protection filter 14 slidably disposed along a guidewire 16. As is discussed in greater detail below, the snare retrieval catheter 12 is configured to ensnare the embolic protection filter 14 and retrieve the device from blood vessel.

Embolic protection filter 14 may comprise a filter frame 18, a plurality of expandable struts 20, and a filter mesh or membrane 22 operatively coupled to the expandable struts 20. The expandable struts 20 are biased to expand in an outward direction when unconstrained radially, allowing the filter mesh or membrane 22 to expand when placed within the vessel. Several openings or pores 24 disposed on the filter mesh or membrane 22 are adapted to capture embolic debris contained in the blood while permitting the perfusion of blood through the vessel.

Filter frame 18 defines a tubular member having a proximal portion 26, a distal portion 28, and an inner lumen (not shown) configured to slidably receive the guidewire 16. The proximal portion 26 of filter frame 18 includes a radial notch 34 forming a collar that can be engaged by the snare retrieval catheter 12 to retrieve the embolic protection filter 14 from the vessel.

In the particular view depicted in Figure 1, the embolic protection filter 14 and snare retrieval catheter 12 are shown advanced towards the distal end 30 of the guidewire 16. The snare retrieval catheter 12 is positioned proximate and proximal the embolic protection filter 14 just prior to retrieval of the device. A distal stop 32 placed at or near the distal end 30 of the guidewire 16 prevents movement of the embolic protection filter 14 beyond the distal end 30 thereof. The distal stop 32 may be formed from a tubular segment having an inner lumen that forms a friction fit when inserted over the guidewire 16. The friction fit prevents movement of the distal stop 32 along the guidewire 16 in the absence of a force exerted by the operator via a push tube or other pushing means.

Snare retrieval catheter 12 comprises an elongated tubular member 36 having a proximal end 38 and a distal end 40. As shown in Figure 2, the elongated tubular member 36 defines a first lumen 42 configured to slidably receive the guidewire 16, and a second lumen 44 configured to receive an elongated member 46 that can be actuated within the snare retrieval catheter 12 to retrieve the embolic protection filter 14. The first and second lumens 42, 44 extend axially through the entire length of the elongated tubular member 36, terminating at the proximal and distal ends 38, 40 thereof.

The elongated tubular member 36 may comprise a hypo-tube formed of a metal, metal-alloy or metal-polymer composite. Examples of suitable materials include stainless steel, nickel-titanium alloy, polyethylene terapthalate (PET), polytetraflouroethylene (PTFE), polyurethane, fluorinated ethylene propylene (FEP), polypropylene (PP), polyvinylchloride (PVC), polyether-ether ketone (PEEK), polyimide, polyester, polyamide, elastomeric polyamides, block polyamide/ethers, polyether block amide (PEBA), and polyetherimide (PEI).

The elongated member 46 may comprise a flexible wire or cord that extends axially through the second lumen 44, out the distal end 40 of the snare retrieval catheter 12, and is then looped-back through the second lumen 44. In the exemplary embodiment illustrated in Figure 1, both ends 48, 50 of the elongated member 46 extend proximal the proximal end 38 of the catheter 12 at a location outside of the patient's body.

The portion of the elongated member 46 extending distally from the distal end 40 of the catheter 12 forms a loop 52, the size of which can be adjusted by moving the elongated member 46 within the second lumen 44. As shown in Figure 1, the loop 52 is bent at an angle relative to the longitudinal axis formed by the catheter 12 such that the opening 54 formed by the loop 52 encircles the guidewire 16. The opening 54 formed by loop 52 is also centrally disposed about a central axis defined by the guidewire 16 such that the loop 52 is concentric with the radial notch 34 formed on the filter frame 18.

To actuate the snare retrieval catheter 12, the loop 52 may be adjusted by pulling one or both of the ends 48, 50, causing the elongated member 46 to move axially within the second lumen 44. The size of the loop 52 can be adjusted to ensnare the radial notch 34 formed on the proximal portion 26 of the filter frame 18. A locking hub or other locking means (not shown) can be utilized to lock one or both of the ends 48, 50 to prevent undesired expansion or contraction of the loop 52 once the embolic protection filter 14 has been ensnared.

In some embodiments, the elongated member 46 can be formed of a metal such as stainless steel or nickel-titanium alloy, or a fibrous polymer such as polyethylene, nylon, polyester, or polyurethane. The elongated member 46 may be formed from several threads or strands woven together to form an axially stiff but radially flexible member. The structure and dimensions of the elongated member 46 can be selected to impart a particular performance characteristic, depending on the particular intravascular device to be retrieved and the particular location of the body to be traversed. For example, to retrieve an embolic protection filter from the distal vasculature, the length of the snare retrieval catheter may approach or even exceed 300 cm in length. Due to the relatively long length of the elongated member 46, buckling or kinking may result when the member 46 is compressed. Moreover, the elongated member 46 may tend to yield or even break if the tensile strength of the member 46 is insufficient to transfer the load of the embolic protection filter 14. Accordingly, the dimensions and/or materials of the elongated member 46 can be selected to account for the tensile, compressive, and torsional stresses resulting from the advancement and/or retraction of the elongated member 46 within the second lumen 44.

In certain embodiments, the elongated member 46 may be formed of a radiopaque material (*e.g*. gold, platinum, tungsten, tantalum), or may include a radiopaque additive (*e.g*. barium sulfate, barium subcarbonate), allowing the operator to determine the location of the elongated member 46 within the body. In use, a contrast media can be injected into the vessel, and a fluoroscopic monitor located outside of the patient's body can be utilized to determine the precise location of the elongated member 46 within the vessel.

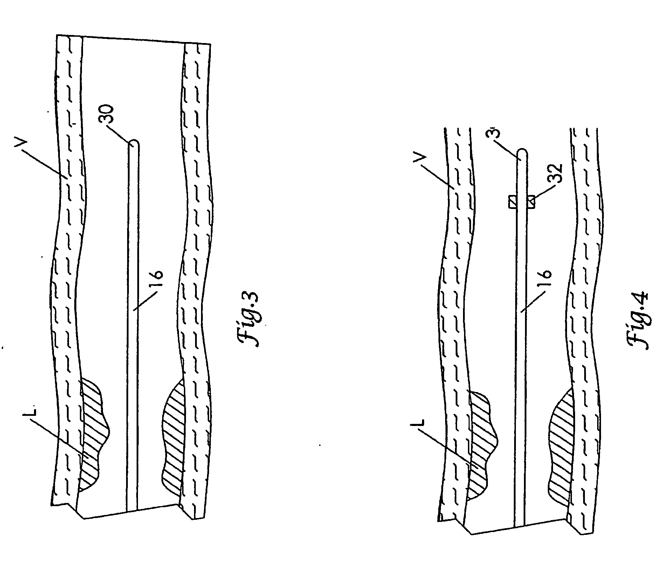

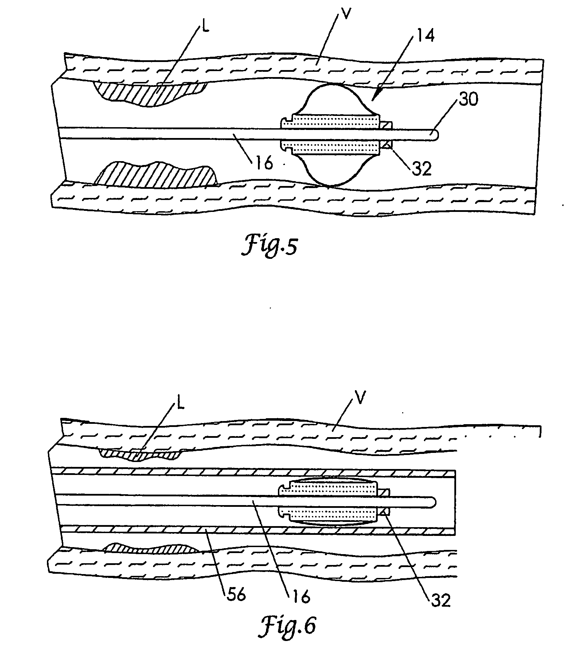

Figures 3-7 depict a retrieval method using the snare retrieval catheter 12 and embolic protection filter 14 described above in the context of a therapeutic procedure such as percutaneous transluminal coronary angioplasty (PTCA). In a first position illustrated in Figure 3, a guiding device such as a guidewire 16 can be advanced through a vessel V, and placed at a desired location distal a lesion L. Once positioned, a distal stop 32 can be placed at or near the distal end 30 of the guidewire 16, as shown in Figure 4. The embolic protection filter 14 can then be advanced along the guidewire 16 and placed at a location proximal the distal stop 32, as shown in Figure 5. The distal stop 32 prevents the operator from advancing the embolic protection filter 14 beyond the distal end 30 of the guidewire 16. An angioplasty procedure can then be conducted upstream of the embolic protection filter 14 to treat the lesion L. At the conclusion of the therapeutic procedure, a sheath 56 can be advanced along the guidewire 16 to collapse the embolic protection filter 14 and its contents therein, as shown in Figure 6.

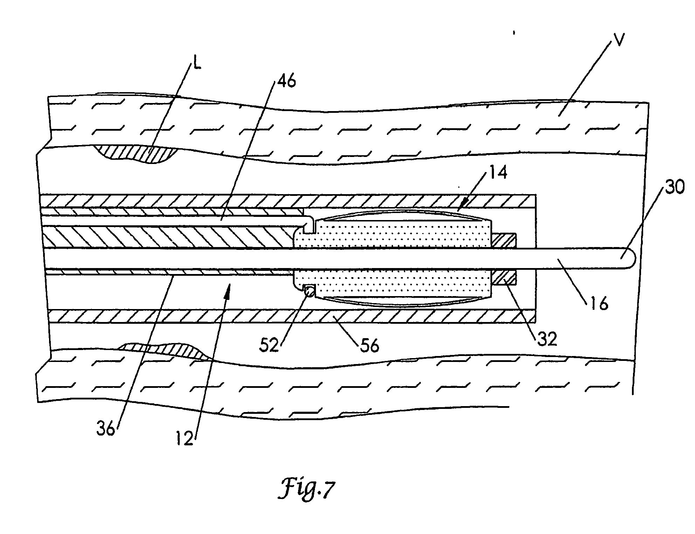

Once the embolic protection filter 14 has been collapsed within the sheath 56, the snare retrieval catheter 12 can be advanced along the guidewire 16 and positioned such that the loop 52 is disposed at least in part about the radial notch 34. Holding the snare retrieval catheter 10 stationary, the operator can then retract the elongated member 46 proximally through the second lumen 44 to reduce the size of the loop 52 and ensnare the embolic protection filter 14, as shown in Figure 7. The sheath 56, snare retrieval catheter 12, and captured embolic protection filter 14 can then be removed from the body.

It should be understood that other intravascular devices can be retrieved using the devices described herein. For example, although a cartridge-type embolic protection filter is illustrated, a basket, umbrella, or hoop-type embolic protection device can be retrieved using the devices and methods described above. Moreover, intravascular devices for use in other areas of the body (e.g. the vena cava) can be retrieved using the devices and methods described herein.

Having thus described the several embodiments of the present invention, those of skill in the art will readily appreciate that other embodiments may be made and used which fall within the scope of the claims attached hereto. Numerous advantages of the invention covered by this document have been set forth in the foregoing description. It will be understood that this disclosure is, in many respects, only illustrative. Changes may be made in details, particularly in matters of shape, size and arrangement of parts without exceeding the scope of the invention.

## Claims

1. A medical retrieval device for an intravascular filter within a blood vessel, comprising:
a guidewire (16);
an embolic protection filters (14) slidably disposed along the guidewire (16), and a snare retrieval catheter (12) apted to ensnare the embolic protection filter (14);
wherein the snare retrieval catheter further comprises:
an elongated tubular member (36) having a proximal end (38), a distal end (40) first lumen (42) configured to slidably receive the guidewire (16), and a second lumen configured to receive an elongated member (46), elongated member having a first end (48) a second end, (50) and a loop portion (52) extending distally of the distal end (40) said elongated tubular member (36) such that said loop portion (52) encircles the guide wire (16) and is centrally disposed about a central axis defined by the guide wire (16) thereby forming an adjustable snare that can be sized within the blood vessel to retrieve the embolic protection filter (14),

2. The medical retrieval device of claim 1, wherein said embolic protection filter (14) comprises a filter frame (18), a plurality of expandable struts (20), and a filter mesh or membrane (22) operatively coupled to said plurality of expandable struts(20).

3. The medical retrieval device of claim 2, wherein said filter frame (18) includes a radial notch (34).

4. The medical retrieval device of claim 1, wherein said elongated members (46) wire.

5. The medical retrieval device of claim 1, wherein said elongated member (46) is a cord.

6. The medical retrieval device of claim 1, wherein the loop portion (52) is bent at an angle relative to the longitudinal axis formed by the elongated tubular member (36).

7. The medical retrieval device of claim 1, further comprising a distal stop (32) disposed about the guidewire (16).

## Patentansprüche

1. Medizinische Bergungsvorrichtung zum Bergen eines intravaskulären Filters in einem Blutgefäß mit:
einem Führungsdraht (16);
einem Embolieprotektionsfilter (14), das entlang dem Führungsdraht (16) verschiebbar angeordnet ist; und
einem Schlingenbergungskatheter (12), der geeignet ist, das Embolieprotektionsfilter (14) zu umschlingen;
wobei der Schlingenbergungskatheter ferner aufweist:
ein längliches Röhrenteil (36) mit einem proximalen Ende (38), einem distalen Ende (40), einem ersten Lumen (42), das so konfiguriert ist, dass es den Führungsdraht (16) verschiebbar aufnimmt, und einem zweiten Lumen, das so konfiguriert ist, dass es ein längliches Teil (46) aufnimmt, wobei das längliche Teil ein erstes Ende (48),
ein zweites Ende (50) und einen Schlaufenabschnitt (52) hat, der sich distal vom distalen Ende (40) des länglichen Röhrenteils (36) so erstreckt, dass der Schlaufenabschnitt (52) den Führungsdraht (16) umschließt und um eine durch den Führungsdraht (16) festgelegte Mittelachse mittig angeordnet ist, wodurch er eine einstellbare Schlinge bildet, die im Blutgefäß so bemessen sein kann, dass sie das Embolieprotektionsfilter (14) birgt.

2. Medizinische Bergungsvorrichtung nach Anspruch 1, wobei das Embolieprotektionsfilter (14) aufweist: einen Filterrahmen (18), mehrere expandierbare Streben (20) und ein Filtermaschenmaterial oder eine Filtermembran (22), die mit den mehreren expandierbaren Streben (20) in Wirkbeziehung gekoppelt ist.

3. Medizinische Bergungsvorrichtung nach Anspruch 2, wobei der Filterrahmen (18) eine Radialkerbe (34) aufweist.

4. Medizinische Bergungsvorrichtung nach Anspruch 1, wobei das längliche Teil (46) ein Draht ist.

5. Medizinische Bergungsvorrichtung nach Anspruch 1, wobei das längliche Teil (46) eine Schnur ist.

6. Medizinische Bergungsvorrichtung nach Anspruch 1, wobei der Schlaufenabschnitt (52) in einem Winkel relativ zu der durch das längliche Röhrenteil (36) gebildeten Längsachse abgebogen ist.

7. Medizinische Bergungsvorrichtung nach Anspruch 1, ferner mit einem distalen Anschlag (32), der um den Führungsdraht (16) angeordnet ist.

## Revendications

1. Dispositif de récupération médical destiné à récupérer un filtre intravasculaire à l'intérieur d'un vaisseau sanguin, comprenant :
un guide-fil (16) ;
un filtre de protection embolique (14) disposé de manière coulissante le long du guide-fil (16) ; et
un cathéter de récupération à collet (12) conçu pour piéger le filtre de protection embolique (14) ;
dans lequel le cathéter de récupération à collet comprend en outre :
un élément tubulaire allongé (36) ayant une extrémité proximale (38), une extrémité distale (40), une première lumière (42) configurée pour recevoir de manière coulissante le guide-fil (16), et une seconde lumière configurée pour recevoir un élément allongé (46), ledit élément allongé ayant une première extrémité (48), une seconde extrémité (50), et une partie de boucle (52) s'étendant distalement de l'extrémité distale (40) dudit élément tubulaire allongé (36) de sorte que ladite partie de boucle (52) encercle le guide-fil (16) et soit disposée au centre autour d'un axe central défini par le guide-fil (16) formant de ce fait un collet ajustable qui peut être dimensionné à l'intérieur du vaisseau sanguin pour récupérer le filtre de protection embolique (14).

2. Dispositif de récupération médical selon la revendication 1, dans lequel ledit filtre de protection embolique (14) comprend un cadre de filtre (18), une pluralité d'entretoises extensibles (20) et une toile ou membrane de filtre (22) couplée de façon opérationnelle à ladite pluralité d'entretoises extensibles (20).

3. Dispositif de récupération médical selon la revendication 2, dans lequel ledit cadre de filtre (18) comprend une encoche radiale (34).

4. Dispositif de récupération médical selon la revendication 1, dans lequel ledit élément allongé (46) est un fil.

5. Dispositif de récupération médical selon la revendication 1, dans lequel ledit élément allongé (46) est un cordon.

6. Dispositif de récupération médical selon la revendication 1, dans lequel la partie de boucle (52) est pliée en formant un angle par rapport à l'axe longitudinal formé par l'élément tubulaire allongé (36).

7. Dispositif de récupération médical selon la revendication 1, comprenant en outre une butée distale (32) disposée autour du guide-fil (16).
